# FASCICULE DE BREVET EUROPEEN

(11) **EP 4 073 172 B1**
(45) Date de publication et mention de la délivrance du brevet: **12.11.2025**
(21) Numéro de dépôt: 20817358.3
(22) Date de dépôt: 07.12.2020
(51) Int. Cl.: A61K 8/06, A61Q 19/00, A61K 9/00, A61K 47/32, C08K 5/17, C08L 33/24, A61K 8/81, C08F 220/58

(54) **PROCEDE DE PREPARATION D'UN LATEX INVERSE POUR COMPOSITION COSMETIQUE COMBINANT DE L' EDDS COMME AGENT SEQUESTRANT ET UN POLYELECTROLYTE COMPRENANT DE L'AMPS ET DE L'ACRYLAMIDE**
VERFAHREN ZUR HERSTELLUNG EINES INVERSEN LATEX FÜR KOSMETISCHE ZUSAMMENSETZUNGEN MIT EDDS ALS SEQUESTRIERMITTEL UND EINEM POLYELEKTROLYTEN, DER AMPS UND ACRYLAMID ENTHÄLT
METHOD FOR PREPARING AN INVERSE LATEX FOR COSMETIC COMPOSITION COMBINING EDDS AS SEQUESTERING AGENT AND A POLYELECTROLYTE COMPRISING AMPS AND ACRYLAMIDE

(30) Priorité: 09.12.2019 FR 1913977
(43) Date de publication de la demande: 19.10.2022
(73) Titulaire: Société d'Exploitation de Produits pour les Industries Chimiques SEPPIC, 75321 Paris cedex 07 (FR)
(72) Inventeur: BODOC, Miruna, 81100 Castres (FR); COLAS, Aurélie, 92250 La Garenne Colombes (FR)
(74) Mandataire: Air Liquide
(86) Numéro de dépôt international: PCT/EP2020/084839
(87) Numéro de publication internationale: WO 2021/116004

(56) Documents cités:
- EP-A1- 3 037 082
- WO-A1-2019/193294
- FR-A1- 2 886 300

## Description

La présente invention est relative à un procédé de préparation d'un latex inverse auto-inversible comprenant un nouvel agent séquestrant. La présente divulgation décrit également ce latex inverse auto-inversible, l'utilisation desdits latex inverses auto-inversibles comme épaississants utilisés pour préparer des compositions cosmétiques ou pharmaceutiques à usage topique, ainsi que lesdites compositions ainsi préparées.

Les polymères sont largement utilisés aujourd'hui dans les formulations cosmétiques à usage topique et représentent la deuxième famille de produits les plus utilisés dans ce type de formulations. Les compositions cosmétiques contiennent des phases polaires comme par exemple des phases constituées d'eau, et nécessitent dans la plupart des cas l'utilisation des polymères modificateur de rhéologie pour augmenter la viscosité de ces phases polaires, ainsi que pour conférer un comportement rhéologique bien défini.

Parmi les polymères modificateurs de rhéologie des phases polaires, nous pouvons citer des polymères naturels ou bien des polymères synthétiques, et notamment de type polyélectrolyte, anionique ou cationique, amphiphile, linéaires ou ramifiés, réticulés ou non réticulés. Ces polymères, une fois introduits dans des phases polaires présentent la propriété de se déployer sous l'effet des répulsions électrostatiques dues à la présence des charges (négatives et/ou positives) sur le squelette polymérique, linéaire ou ramifié, non réticulé ou réticulé. Les modificateurs de rhéologie apportent à la fois une augmentation de la viscosité de la phase polaire, ainsi qu'une certaine consistance et/ou un effet stabilisant de la formule cosmétique, dermo-cosmétique ou dermopharmaceutique à épaissir.

Afin de répondre aux besoins des consommateurs et d'améliorer les formulations cosmétiques à usage topique, les scientifiques ont mis au point de nouveaux systèmes polymériques innovants et variés. Ainsi, les polymères utilisés dans la cosmétique à usage topique ou la dermocosmétique peuvent jouer un rôle en tant qu'agents filmogènes, modificateurs de rhéologie, permettre la stabilisation des phases grasses dans les émulsions (de type eau-dans-huile ou huile-dans-eau) ou la stabilisation de particules (pigments ou charges), ou encore conférer des propriétés sensorielles particulières après application sur la peau (comme par exemple douceur au toucher, facilité de préhension et d'application, effet fraîcheur, etc), ayant également un impact direct sur l'aspect de la formule (translucide ou opaque).

Les polymères modificateurs de rhéologie de phase aqueuse, principalement des polyélectrolytes, résultent de la polymérisation radicalaire de monomères de type (méth)acrylate, c'est-à-dire d'esters dérivés de l'acide acrylique ou de l'acide méthacrylique, ou encore des dérivés d'acrylamide.

Aujourd'hui ces polymères, pouvant se présenter sous forme de latex inverse, de latex inverse concentré, ou de poudre permettent de répondre aux besoins des clients en terme de performances épaississantes, dans un solvant polaire, comme l'eau par exemple. Les gels aqueux obtenus une fois ces polymères dispersés dans l'eau présentent un aspect lisse, exempt de grains ou grumeaux, avec des propriétés sensorielles particulières au toucher, ainsi qu'une facilité de préhension et d'application.

La forme liquide, connu sous le nom "latex inverse auto-inversible", ou sa forme liquide concentrée, est une composition se présentant sous forme d'émulsion eau-dans-huile et comprend:
- une phase aqueuse, elle-même comprenant au moins un polymère de type polyélectrolyte, de type anionique, ou cationique, ou ampholyte, linéaire et/ou branché et/ou réticulé
- une phase grasse comprenant au moins une huile
- au moins un tensioactif émulsionnant (S₁) de type eau-dans-huile,
- au moins un tensioactif émulsionnant (S₂) de type huile-dans-eau,
ledit polymère étant obtenu par la mise en œuvre d'un procédé de polymérisation radicalaire en émulsion inverse comme décrit dans la demande internationale WO 2019/193294 A1.

La polymérisation radicalaire est connue pour sa sensibilité à la présence d'impuretés même en faibles quantités. Des composés qui peuvent induire une décroissance de la vitesse de polymérisation à faible concentration sont connus comme des inhibiteurs ou retardateurs. Néanmoins la distinction entre ces deux effets n'est pas toujours simple, et le même composé peut avoir les deux contributions néfastes selon sa concentration dans le milieu ou la nature des monomères et du milieu réactionnel. Des performances reproductibles des polymères épaississants de phases aqueuse doivent être garanties afin d'assurer une qualité constante des formulations cosmétiques à usage topique contenant ces polymères. Pour cela, les fabricants doivent s'assurer que les réactions de polymérisations suivent de façon répétable la même cinétique, plus particulièrement au niveau de la durée d'inhibition, l'exothermie de réaction (°C/min), et de la durée totale de la réaction de polymérisation dans le temps. Etant donné ces contraintes, une attention particulière est attribuée aux facteurs pouvant influencer le démarrage de la réaction de polymérisation radicalaire, par exemple la présence de l'oxygène qui peut retarder la réaction de polymérisation en réagissant avec les radicaux générés. Ces nouveaux radicaux peroxyde présente une réactivité plus faible, la capacité d'initiation étant diminuée. Ceci se traduit par une étape d'initiation et une vitesse de propagation plus faible, donc in fine aboutissant à des polymères ayant des propriétés épaississantes différentes. Une étape de désoxygénation du milieu, notamment par une purge avec de l'azote avant démarrage de la réaction de polymérisation s'avère ainsi nécessaire.

Un autre facteur impactant directement la polymérisation est la présence d'espèces métalliques (Fe²⁺, Fe³⁺, Cu²⁺,...) qui, à leur tour génèrent un effet d'inhibition. Dans ce cas, l'inhibition peut se produire lors de la phase d'amorçage par la réaction des radicaux amorceurs avec des impuretés métalliques, de sorte que le centre radicalaire actif devient alors incapable de fixer une autre unité monomérique et devient inactif lors de la polymérisation.

Les ions métalliques cités précédemment peuvent potentiellement provenir des matières premières ou bien des installations.

Les monomères utilisés pour la préparation de latex inverses auto-inversibles peuvent présenter des traces de cations métalliques. De la même façon, il n'est pas impossible d'envisager la présence de contaminants métalliques dans les installations industrielles accueillant les réactions de polymérisation. Dans la plupart des cas, les installations sont réalisées en acier inoxydable (couramment appelé inox ou acier inox), et nous retrouvons plusieurs types d'acier inoxydable qui diffèrent selon leur composition. L'inox est un alliage à base de fer, additionné de nickel, de chrome, ou de molybdène dans certains cas. C'est le chrome qui confère à l'inox ses propriétés anti-oxydantes, car en présence d'oxygène, il est capable de régénérer tout seul sa couche d'oxyde de chrome de surface appelée couche passive.

Néanmoins, il n'est pas impossible qu'en contact prolongé avec des sources de pollution, acides, humidité, embruns, poussières chargées en fer, ou en cas de rayures profondes, la couche de protection va alors se dé-passiver (donc s'activer) et l'inox va s'oxyder plus vite qu'il sera capable de se protéger. Dans ces cas, nous pouvons retrouver l'apparition de la rouille, donc une source de contaminants métalliques à base de fer.

Compte tenu des risques associés à la présence de toutes ces sources de contaminants métalliques, l'utilisation d'un agent séquestrant est incontournable. Le produit généralement utilisé est le sel pentasodique de l'acide diethylenetriaminepentaacetic (connu également sous le nom commercial de Versenex^{™} 80).

Cependant l'évolution de la réglementation européenne concernant la classification du sel pentasodique de l'acide diethylenetriaminepentaacetic nous amène à rechercher une solution alternative comme agent séquestrant pour la préparation de latex inverses auto-inversibles.

Partant de là, un problème qui se pose est de fournir un nouveau latex inverse avec un nouvel agent sequestrant aussi efficace que le sel pentasodique de l'acide diethylenetriaminepentaacetic présentant des propriétés plus en conformité avec l'évolution de la réglementation.

Une solution de la présente invention est un procédé de préparation d'un latex inverse auto-inversible comprenant une phase aqueuse comprenant:
a. un polyélectrolyte anionique réticulé (P) constitué pour 100% molaire de:
   - une proportion entre 20% et 90% d'une unité monomérique issue de l'acide 2-méthyl-2-[(1-oxo-2-propényl) amino] 1-propanesulfonique sous forme d'acide libre ou partiellement ou totalement salifiée;
   - une proportion entre 10% et 80% d'une unité monomérique issue d'au moins un monomère choisi parmi les éléments du groupe constitué par l'acrylamide, le N,N-diméthyl acrylamide, le méthacrylamide, le N-isopropyl acrylamide, le N-tertio-butyl acrylamide; et
   - une proportion supérieure à 0% molaire et inférieure ou égale à 1% molaire d'une unité monomérique issue d'au moins un monomère de réticulation (AR) polyéthylénique choisi parmi le méthylène-bis(acrylamide), le diméthacrylate d'éthylène glycol, le diacrylate de diéthylèneglycol, le diacrylate d'éthylène glycol, le diallyl urée, le triallylamine, le triméthylol propanetriacrylate, l'acide diallyoxyacétique ou un de ses sels comme le diallyloxyacétate de sodium, ou un mélange de ces composés,
b. de l'éthylenediamine disuccinique acide sous forme de sel trisodique, ledit procédé comprenant les étapes suivantes:
   a) Préparation de la phase aqueuse telle que définie ci-dessus,
   b) Préparation d'une phase organique comprenant au moins une huile et un système tensioactif émulsionnant (S1 ) de type eau-dans-huile,
   c) Mélange de la phase aqueuse et de la phase organique préparées aux étapes a) et b) et émulsification de manière à former une émulsion,
   d) Inertage de l'émulsion à l'azote,
   e) Amorçage de la réaction de polymérisation par introduction dans l'émulsion inertée d'un initiateur de radicaux libres, et
   f) Introduction dans le milieu réactionnel issu de l'étape e) d'un système tensioactif émulsionnant (S2 ) de type huile-dans-eau à une température comprise entre 30 et 60°C.

Selon le cas, le procédé de préparation du latex inverse auto-inversible selon l'invention peut présenter une ou plusieurs des caractéristiques suivantes :
- la phase aqueuse comprend au moins 0,01% molaire de l'éthylenediamine disuccinique acide sous forme de sel trisodique.
- le monomère de réticulation (AR) est du méthylène bis(acrylamide) ou du triallylamine.
- le polyélectrolyte anionique réticulé comprend :
   - une proportion entre 20% et 80%, plus particulièrement entre 32% et 70% massique de l'unité monomérique issue de l'acide 2-méthyl-2-[(1-oxo-2-propényl) amino] 1-propanesulfonique sous forme d'acide libre ou partiellement ou totalement salifiée ;
   - une proportion entre 20% et 80%, plus particulièrement entre 30% et 68% massique de l'unité monomérique issue d'au moins un monomère choisi parmi les éléments du groupe constitué par l'acrylamide, le N,N-diméthyl acrylamide, le méthacrylamide, le N-isopropyl acrylamide, le N-tertio-butyl acrylamide, et
   - une proportion molaire inférieure ou égale à 0,5%, particulièrement inférieure ou égale à 0,25%, plus particulièrement inférieure ou égales à 0,1%, et plus particulièrement supérieure ou égales à 0,005% molaire d'unités monomériques issues d'au moins un monomère de réticulation (AR) polyéthylénique.

Au sens de la présente invention, par polyélectrolyte anionique réticulé (P), on désigne pour le polymère (P), un polyélectrolyte non linéaire qui se présente à l'état d'un réseau tridimensionnel insoluble dans l'eau, mais gonflable à l'eau et conduisant alors à l'obtention d'un gel chimique.

Au sens de la présente invention, le terme «salifié» indique que la fonction acide présente dans un monomère se trouve sous une forme anionique associée sous forme de sel à un cation, notamment les sels de métaux alcalins, tels que les cations du sodium ou du potassium, ou comme les cations de base azotés tels que le sel d'ammonium, le sel de lysine ou le sel de monoéthanolamine (HOCH2-CH2-NH3⁺). Il s'agit de préférence des sels de sodium ou d'ammonium.

Selon un aspect particulier du procédé de la présente invention, ledit latex inverse auto-inversible tel que défini ci-dessus comprend de 20% massique à 90% massique, et plus particulièrement de 30% massique à 90% massique, plus particulièrement de 30% massique à 80% massique, et encore plus particulièrement de 33% massique à 80% massique dudit polyélectrolyte anionique (P) réticulé.

Selon un autre aspect particulier du procédé de la présente invention, la proportion molaire en unités monomériques issues de acide 2-méthyl 2-[(1-oxo 2-propènyl) amino] 1-propanesulfonique sous forme acide libre ou partiellement ou totalement salifiée présentes dans ledit polyélectrolyte anionique réticulé (P) est supérieure ou égale à 32% molaire et inférieure ou égale à 100% molaire, plus particulièrement supérieure ou égale à 40% molaire et inférieure ou égale à 100% molaire.

Selon un aspect particulier de la présente invention, l'acide 2-méthyl 2-[(1-oxo 2-propènyl) amino] 1-propanesulfonique est sous forme de sel de de sodium ou d'ammonium.

Selon le cas le procédé selon l'invention peut présenter une ou plusieurs des caractéristiques ci-dessous :
- A l'étape e) l'initiateur de radicaux est un couple oxydo-réducteur générateur d'ions hydrogénosulfite (HSO3-), tel que le couple hydroperoxyde de cumène -métabisulfite de sodium (Na2S2O5) ou le couple hydroperoxyde de cumène-chlorure de thionyle (SOCl2).
- A l'étape e) on introduit dans l'émulsion inertée un co-initiateur de polymérisation, de préférence de l'azo-bis(isobutyronitrile).
- A l'étape a) le pH de la phase aqueuse est ajusté entre 3,0 et 7,0, plus particulièrement entre 3,5 et 6,5, encore plus particulièrement entre 4,0 et 6,5
- le milieu réactionnel issu de l'étape e) est concentré par distillation avant la mise en œuvre de l'étape f).
- le milieu réactionnel issu de l'étape e) ou f) est séché par atomisation.

Par huile (H), on désigne dans la définition dudit latex inverse auto-inversible, notamment :
- Les alcanes linéaires comportant de onze à dix-neuf atomes de carbone ;
- Les alcanes ramifiés, comportant de sept à quarante atomes de carbone, comme l'isododécane, l'isopentadécane, l'isohexadécane, l'isoheptadécane, l'isooctadécane, l'isononadécane ou l'isoeicosane), ou des mélanges de certains d'entre eux comme ceux cités ci-après et identifiés par leur nom INCI : C₇₋₈ isoparaffin, C₈₋₉ isoparaffin, C₉₋₁₁ isoparaffin, C₉₋₁₂ isoparaffin, C₉₋₁₃ isoparaffin, C₉₋₁₄ isoparaffin, C₉₋₁₆ isoparaffin, C₁₀₋₁₁ isoparaffin, C₁₀₋₁₂ isoparaffin, C₁₀₋₁₃ isoparaffin, C₁₁₋₁₂ isoparaffin, C₁₁₋₁₃ isoparaffin, C₁₁₋₁₄ isoparaffin, C₁₂₋₁₄ isoparaffin, C₁₂₋₂₀ isoparaffin, C₁₃₋₁₄ isoparaffin, C₁₃₋₁₆ isoparaffin ;
- Les cyclo-alcanes optionnellement substitués par un ou plusieurs radicaux alkyles linéaires ou ramifies ;
- Les huiles blanches minérales, comme celles commercialisées sous les noms suivants : Marcol^{™}52, Marcol^{™}82, Drakeol^{™}6VR, Eolane^{™}130, Eolane^{™}150 ;
- L'hémisqualane (ou 2,6,10-trimethyl- dodécane ; numéro CAS : 3891-98-3), le squalane (ou 2,6,10,15,19,23-hexamethyltetracosane), le polyisobutène hydrogéné ou le polydécène hydrogéné ;
- Les mélanges d'alcanes comportant de 15 à 19 atomes de carbone, lesdits alcanes étant des alcanes linéaires, des alcanes ramifiés et des cyclo-alcanes, et plus particulièrement le mélange (M₁) qui comprend pour 100% de sa masse, une proportion massique en alcanes ramifiés supérieure ou égale à 90% et inférieure ou égale à 100% ; une proportion massique en alcanes linéaires supérieure ou égale à 0% et inférieure ou égale à 9%, et plus particulièrement inférieure à 5% et une proportion massique en cyclo-alcanes supérieure ou égale à 0% et inférieure ou égale à 1%, par exemple les mélanges commercialisés sous les noms Emogreen^{™}L15 ou Emogreen^{™}L19 ;
- Les éthers d'alcool gras de formule (IV) :

   Z₁-O-Z₂ (IV),

   dans laquelle Z₁ et Z₂ identiques ou différents, représentent un radical alkyle linéaire ou ramifié comportant de cinq à dix-huit atomes de carbone, par exemple les dioctyl éther, didécyl éther, didodécyl éther, dodécyl octyl éther, dihexadécyl éther, (1,3-diméthyl butyl) tétradécyl éther, (1,3-diméthyl butyl) hexadécyl éther, le bis(1,3-diméthyl butyl) éther ou le dihexyl éther.
- Les mono-esters d'acides gras et d'alcools de formule (V) :

   R'₁-(C=O)-O-R'₂ (V),

   dans laquelle R'₁-(C=O) représente un radical acyle, saturé ou insaturé, linéaire ou ramifié, comportant de huit à vingt-quatre atomes de carbone, et R'2 représente, indépendamment de R'₁, une chaîne hydrocarbonée saturée ou insaturée, linéaire ou ramifiée comportant de un à vingt-quatre atomes de carbone, par exemple les laurate de méthyle, laurate d'éthyle, laurate de propyle, laurate d'isopropyle, laurate de butyle, laurate de 2-butyle, laurate d'hexyle, cocoate de méthyle, cocoate d'éthyle, cocoate de propyle, cocoate d'isopropyle, cocoate de butyle, cocoate de 2-butyle, cocoate d'hexyle, myristate de méthyle, myristate d'éthyle, myristate de propyle, le myristate d'isopropyle, le myristate de butyle, le myristate de 2-butyle, le myristate d'hexyle, le myristate d'octyle, le palmitate de méthyle, le palmitate d'éthyle, le palmitate de propyle, le palmitate d'isopropyle, le palmitate de butyle, le palmitate de 2-butyle, le palmitate d'hexyle, le palmitate d'octyle , l'oléate de méthyle, l' oléate d'éthyle, l'oléate de propyle, l'oléate d'isopropyle, l'oléate de butyle, l'oléate de 2-butyle, l'oléate d'hexyle, l'oléate d'octyle, le stéarate de méthyle, le stéarate d'éthyle, le stéarate de propyle, le stéarate d'isopropyle, le stéarate de butyle, le stéarate de 2-butyle, le stéarate d'hexyle, le stéarate d'octyle, l'isostéarate de méthyle, l'isostéarate d'éthyle, l'isostéarate de propyle, l'isostéarate d'isopropyle, l'isostéarate de butyle, l'isostéarate de 2-butyle, l'isostéarate d'hexyle, l'isostéarate d'isostéaryle ;
- Les di-esters d'acides gras et de glycérol de formule (VI) et de formule (VII) :

   R'₃-(C=O)-O-CH₂-CH(OH)-CH₂-O-(C=O)-R'₄ (VI)

   R'₅-(C=O)-O-CH₂-CH[O-(C=O)-R'₆]-CH₂-OH (VII),

   formules (VI) (VII) dans lesquelles R'₃-(C=O), R'₄-(C=O), R'₅-(C=O), R'₆-(C=O), identiques ou différents, représentent un groupement acyle, saturé ou insaturé, linéaire ou ramifié, comportant de huit à vingt-quatre atomes de carbone ;
- Les tri-esters d'acides gras et de glycérol de formule (VIII) :

   R'₇-(C=O)-O-CH₂-CH[O-(C=O)-R"₈]-CH₂-O-(C=O)-R"₉ (VIII),

   dans laquelle R'₇-(C=O), R'₈-(C=O) et R'₉-(C=O), identiques ou différents, représentent un groupement acyle, saturé ou insaturé, linéaire ou ramifié, comportant de huit à vingt-quatre atomes de carbone.

Selon un autre aspect particulier de la présente invention, ladite huile (H) est choisie parmi l'undécane, le tridécane, l'isododécane ou l'isohexadécane, les mélanges d'alcanes et d'isoalcanes et de cycloalcanes comme le mélange (M₁) tel que défini précédemment et les mélanges commercialisés sous les noms Emogreen^{™}L15, Emogreen^{™}L19, Emosmart^{™}L15, Emosmart^{™}L19, Emosmart^{™}V21, Isopar^{™}L ou Isopar^{™}M ; les huiles blanches minérales commercialisées sous les noms Marcol^{™}52, Marcol^{™}82, Drakeol^{™}6VR, Eolane^{™}130 ou Eolane^{™}150 ; l'hémisqualane, le squalane, le polyisobutène hydrogéné ou le polydécène hydrogéné ; le dioctyl éther ou le didécyl éther ; le myristate d'isopropyle, le palmitate d'hexyle, le palmitate d'octyle, l'isostéarate d'isostéaryle, l'octanoyl/décanoyl triglycéride, l'hexadécanoyl/octadécanoyl triglycéride, les triglycérides issus de l'huile de colza, de l'huile de tournesol, de l'huile de lin ou de l'huile de palme.

Dans le procédé de préparation du latex inverse auto-inversible objet de la présente invention, le système émulsionnant (S₁) de type eau-dans-huile est constitué soit d'un seul tensioactif émulsionnant soit d'un mélange de tensioactifs émulsionnants, à condition que ledit système tensioactif émulsionnant (S₁) résultant ait une valeur de HLB suffisamment faible pour induire la formation d'émulsions de type eau-dans-huile.

Comme tensioactif émulsionnant (S₁) de type eau-dans-huile, il y a par exemple les esters d'anhydro hexitol et d'acides carboxyliques aliphatiques, linéaires ou ramifiés, saturés ou insaturés, comportant de 12 à 22 atomes de carbone éventuellement substitués avec un ou plusieurs groupes hydroxyles, et plus particulièrement les esters d'anhydro hexitol choisis parmi les anhydro-sorbitols et les anhydro-mannitols et d'acides carboxyliques aliphatiques, linéaires ou ramifiés, saturés ou insaturés, comportant de 12 à 22 atomes de carbone éventuellement substitués avec un ou plusieurs groupes hydroxyles.

Selon un autre aspect particulier du procédé de la présente invention, ledit système tensioactif émulsionnant (S₁) de type eau-dans-huile est choisi parmi les éléments du groupe constitué par le laurate de sorbitan, par exemple celui commercialisé sous le nom Montane^{™}20, le palmitate de sorbitan, par exemple celui commercialisé sous le nom Montane^{™}40, le stéarate de sorbitan, par exemple celui commercialisé sous le nom Montane^{™}60, l'oléate de sorbitan, par exemple celui commercialisé sous le nom Montane^{™}80, le sesquioléate de sorbitan, par exemple celui commercialisé sous le nom Montane^{™}85, le trioléate de sorbitan, par exemple celui commercialisé sous le nom Montane^{™}83, l'isolaurate de sorbitan, l'isostéarate de sorbitan, par exemple celui commercialisé sous le nom Montane^{™}70, le laurate de mannitan, l'oléate de mannitan, ou un mélange de ces esters ; les polyesters de poids moléculaire compris entre 1000 et 3000 et issus de la condensation entre un acide poly(isobutényl) succinique ou son anhydride, tels que l'HYPERMER^{™} 2296, ou le mélange commercialisé sous le nom de marque SIMALINE^{™}IE 501 A, les polyhydroxystéarates de polyglycols de formule (IX) : 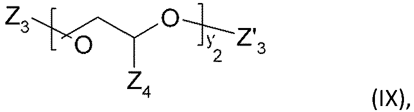 formule (IX) dans laquelle y₂ représente un nombre entier supérieur ou égal à 2 et inférieur ou égal à 50, Z₄ représente l'atome d'hydrogène, le radical méthyle, ou le radical éthyle, Z₃ représente un radical de formule (X) : 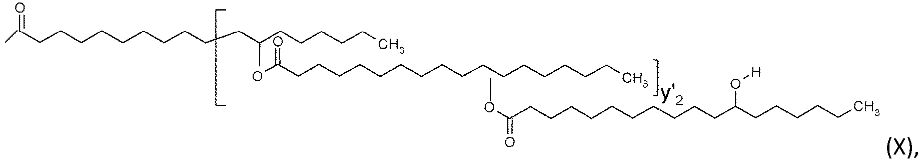 formule (X) dans laquelle y'₂ représente un nombre entier supérieur ou égal à 0 et inférieur ou égal à 10, plus particulièrement supérieur ou égal à 1 et inférieur ou égal à 10 et Z'₃ représente un radical de formule (X) telle que définie ci-dessus, avec Z₃' identique ou différent de Z₃, ou l'atome d'hydrogène.

Comme exemple de tensioactif émulsionnant de type eau-dans-huile de formule (IX) que l'on peut utiliser pour préparer le système émulsionnant (S₁), il y a le PEG-30 dipolyhydroxystéarate commercialisé sous le nom SIMALINE^{™} WO, ou bien les mélanges comprenant le PEG-30 dipolyhydroxystéarate et commercialisés sous les noms SIMALINE^{™}IE 201 A et SIMALINE^{™}IE 201 B, ou encore le mélange comprenant du Triméthylolpropane-30 tripolyhydroxystéarate commercialisé sous le nom SIMALINE^{™}IE 301 B.

Selon un aspect particulier du procédé de l'invention, le système émulsionnant de type huile-dans-eau (S₂) comprend pour 100% de sa masse une proportion supérieure ou égale à 50% massique et inférieure ou égal à 100% d'une composition (Ce) qui comprend pour 100% de sa masse :
- De 10% massique à 60% massique, plus particulièrement de 15% massique à 60% massique et tout particulièrement de 15% massique à 50% d'au moins un composé de formule (I) :

   HO-[CH₂-CH(OH)-CH₂-O]ₙ-H (I)

   dans laquelle n représente un nombre entier supérieur ou égal à un et inférieur ou égal à quinze ;
- De 40% massique à 90 % massique, plus particulièrement de 40% massique à 85 % massique et tout particulièrement de 50% massique à 85% massique d'au moins un composé de formule (II) :

   R₁-(C=O)-[O-CH₂-CH(OH)-CH₂]ₚ-OH (II),

   dans laquelle p, différent ou identique à n, représente un nombre entier supérieur ou égal à un et inférieur ou égal à quinze ; et dans laquelle le groupe R₁-(C=O)- représente un radical aliphatique saturé ou insaturé, linéaire ou ramifié, comportant de six à vingt-deux atomes de carbone, et optionnellement
- Jusqu'à 30% massique, plus particulièrement de 0% massique à 25% massique et tout particulièrement de 0% massique à 20% massique d'au moins une composition (C₁₁) représentée par la formule (III) :

   HO-[CH₂-CHOH-CH₂-O-]_{q}-(G)ᵣ-H (III),

   dans laquelle q différent ou identique à n, représente un nombre entier supérieur ou égal à un et inférieur ou égal à 3, G représente le reste d'un sucre réducteur et r représente un nombre décimal supérieur ou égal à 1,05 et inférieur ou égal à 5,00,
ladite composition (C₁₁) consistant en un mélange des composés de formules (III₁), (III₂), (III₃), (III₄) et (III₅) :

HO-[CH₂-CHOH-CH₂-O-]_{q} -O-(G)₁-H (III₁),

HO-[CH₂-CHOH-CH₂-O-]_{q} -O-(G)₂-H (III₂),

HO-[CH₂-CHOH-CH₂-O-]_{q} -O-(G)₃-H (III₃),

HO-[CH₂-CHOH-CH₂-O-]_{q} -O-(G)₄-H (III₄),

HO-[CH₂-CHOH-CH₂-O-]_{q} -O-(G)₅-H (III₅),

en des proportions molaires en dits composés de formules (III₁), (III₂), (III₃), (III₄) et (III₅) respectivement égales à a₁, a₂, a₃, a₄ et a₅, telles que la somme (a₁+ a₂ + a₃ + a₄ + a₅) est égale à un, et que la somme (a₁ + 2a₂ + 3a₃ + 4a₄ + 5a₅) est égale à r.

Le système émulsionnant (S2) de type huile-dans-eau est constitué soit de la seule composition (Ce), soit d'un mélange de ladite composition (Ce) avec un ou plusieurs autres tensioactifs émulsionnants, à condition que ledit système émulsionnant (S2) résultant ait une valeur de HLB suffisamment élevée pour induire la formation d'émulsions de type huile-dans-eau.

Par sucre réducteur, on désigne dans la formule (III) telle que définie précédemment, les dérivés saccharidiques qui ne présentent pas dans leurs structures de liaison glycosidique établie entre un carbone anomérique et l'oxygène d'un groupement acétal tels qu'ils sont définis dans l'ouvrage de référence : "Biochemistry", Daniel Voet/Judith G. Voet, p. 250, John Wyley & Sons, 1990. La structure oligomérique (G)x peut se présenter sous toutes formes d'isoméries, qu'il s'agisse d'isomérie optique, d'isomérie géométrique ou d'isomérie de position ; elle peut aussi représenter un mélange d'isomères.

Concernant la réaction de polymérisation, celle-ci est amorcée à l'étape e) à un température préférentielle de 10°C, puis conduite soit de manière quasi adiabatique jusqu'à une température supérieure ou égale à 50° C, soit en contrôlant la température.

La présente divulgation a aussi pour objet l'utilisation dudit latex inverse auto-inversible tel que défini précédemment, comme agent épaississant et/ou émulsionnant et/ou stabilisant d'une composition topique cosmétique à usage topique ou pharmaceutique. L'invention a également pour objet une composition cosmétique topique (F) ou une composition pharmaceutique topique (G), caractérisée en ce qu'elle comprend comme agent épaississant, pour 100% de sa masse totale entre 0,1% et 10% massique dudit latex inverse auto-inversible tel que défini précédemment.

L'expression "topique" utilisée dans les définitions des dites compositions (F) et (G), signifie qu'elles sont mises en œuvre par application sur la peau, les cheveux, le cuir chevelu ou les muqueuses, qu'il s'agisse d'une application directe dans le cas d'une préparation cosmétique, dermocosmétique, dermopharmaceutique ou pharmaceutique ou d'une application indirecte par exemple dans le cas d'un produit de soin corporel sous forme de lingette en textile ou en papier ou de produits sanitaires destinés à être en contact avec la peau ou les muqueuses.

Lesdites compositions (F) et (G), se présentent généralement sous forme d'une solution aqueuse ou hydro-alcoolique ou hydro-glycolique, sous forme d'une suspension, d'une émulsion, d'une microémulsion ou d'une nano-émulsion, qu'elles soient de type eau-dans- huile, huile-dans-eau, eau-dans-huile-dans-eau ou huile-dans-eau-dans-huile.

Lesdites compositions (F) et (G), peut être conditionnée dans un flacon, dans un dispositif de type "flacon" pompe, sous forme pressurisées dans un dispositif aérosol, dans un dispositif muni d'une paroi ajourée comme une grille ou dans un dispositif muni d'un applicateur à billes (dit "roll-on").

De façon générale, lesdites compositions (F) et (G) comportent également des excipients et ou des principes actifs habituellement mis en œuvre dans le domaine des formulations à usage topique, en particulier cosmétiques, dermocosmétiques, pharmaceutiques ou dermopharmaceutiques, comme les tensioactifs épaississants et/ou gélifiants, les stabilisants, les composés filmogènes, les agents hydrotropes, les agents plastifiants, les agents émulsionnants et co-émulsionnants, les agents opacificants, les agents nacrants, les agents surgraissants, les séquestrants, les agents chélatants, les antioxydants, les parfums, les conservateurs, les agents conditionneurs, les agents blanchissants destinés à la décoloration des poils et de la peau, les principes actifs destinés à apporter une action traitante vis à vis de la peau ou des cheveux, les filtres solaires, les charges minérales ou les pigments, les particules procurant un effet visuel ou destinées à l'encapsulation d'actifs, les particules exfoliantes, les agents de texture.

Comme exemples de tensioactifs moussants et/ou détergents que l'on peut associer au dit latex inverse auto-inversible tel que défini précédemment dans lesdites compositions (F) et (G), il y a les tensioactifs moussants et/ou détergents anioniques, cationiques, amphotères ou non ioniques.

Parmi les tensioactifs anioniques moussants et/ou détergents que l'on peut associer au dit latex inverse auto-inversible tel que défini précédemment dans lesdites compositions (F) et (G), il y a les sels de métaux alcalins, de métaux alcalino-terreux, d'ammonium, d'amines, ou d'aminoalcools d'alkylether sulfates, d'alkyl sulfates, d'alkylamidoéther sulfates, d'alkylaryl polyéthersulfates, de monoglycérides sulfates, d'alphaoléfinesulfonates, de paraffines sulfonates, d'alkyl phosphates, d'alkyléther phosphates, d'alkyl sulfonates, d'alkylamide sulfonates, d'alkylaryl sulfonates, d'alkyl carboxylates, d'alkyl sulfosuccinates, d'alkyléther sulfosuccinates, d'alkylamide sulfosuccinates, d'alkyl sulfoacétates, d'alkyl sarcosinates, d'acyl iséthionates, de N-acyl taurates, d'acyl lactylates, de dérivés N-acylés d'acides aminés, de dérivés N-acylés de peptides, de dérivés N-acylés de protéines, de dérivés N-acylés d'acides gras. Parmi les tensioactifs amphotères moussants et/ou détergents que l'on peut associer au dit latex inverse auto-inversible tel que défini précédemment dans lesdites compositions (F) et (G), il y a les alkylbétaïnes, les alkylamidobétaïnes, les sultaïnes, les alkylamidoalkylsulfobétaïnes, les dérivés d'imidazolines, les phosphobétaïnes, les amphopolyacétates et les amphopropionates. Parmi les tensioactifs cationiques moussants et/ou détergents que l'on peut associer au dit latex inverse auto-inversible tel que défini précédemment dans lesdites compositions (F) et (G), il y a particulièrement les dérivés d'ammoniums quaternaires. Parmi les tensioactifs non ioniques moussants et/ou détergents que l'on peut associer au dit latex inverse auto-inversible tel que défini précédemment dans lesdites compositions (F) et (G), il y a plus particulièrement les alkylpolyglycosides comportant un radical aliphatique, linéaire ou ramifié, saturé ou insaturé, et comportant de 8 à 16 atomes de carbone, comme l'octyl polyglucoside, le décyl polyglucoside, l'undécylényl polyglucoside, le dodécyl polyglucoside, le tétradécyl polyglucoside, l'hexadécyl polyglucoside, le 1-12 dodécanediyl polyglucoside ; les dérivés d'huile de ricin hydrogénée éthoxylés comme le produit commercialisé sous le nom INCI « Peg-40 hydrogenated castor oil » ; les polysorbates comme le Polysorbate 20, le Polysorbate 40, le Polysorbate 60, le Polysorbate 70, le Polysorbate 80, le Polysorbate 85 ; les amides de coprah ; les N-alkylamines.

Comme exemples de tensioactifs épaississants et/ou gélifiants que l'on peut associer au dit latex inverse auto-inversible tel que défini précédemment dans lesdites compositions (F) et (G), il y a les esters gras d'alkylpolyglycosides éventuellement alcoxylés, comme les esters de méthylpolyglucoside éthoxylés tels que le PEG 120 méthyl glucose trioléate et le PEG 120 méthyl glucose dioléate commercialisés respectivement sous les appellations GLUCAMATE^{™} LT et GLUMATE^{™} DOE120 ; les esters gras alcoxylés tels que le PEG 150 pentaérythrytyl tétrastéarate commercialisé sous l'appellation CROTHIX^{™} DS53, le PEG 55 propylène glycol oléate commercialisé sous l'appellation ANTIL^{™} 141 ; les carbamates de polyalkylène glycols à chaînes grasses comme le PPG-14 laureth isophoryl dicarbamate commercialisé sous l'appellation ELFACOS^{™} T211, le PPG-14 palmeth-60 hexyl dicarbamate commercialisé sous l'appellation ELFACOS^{™} GT2125.

Comme exemples d'agents épaississants et/ou gélifiants que l'on peut associer au dit latex inverse auto-inversible tel que défini précédemment dans lesdites compositions (F) et (G), il y a les copolymères de l'AMPS et d'acrylates d'alkyle dont la chaîne carbonée comprend entre quatre et trente atomes de carbone et plus particulièrement entre dix et trente atomes de carbone, les terpolymère linéaire, branché ou réticulé d'au moins un monomère possédant une fonction acide fort, libre, partiellement salifiée ou totalement salifiée, avec au moins un monomère neutre, et au moins un monomère de formule (XIII) :

CH₂=C(R'₃)-C(=O)-[CH₂-CH₂-O]n'-R'₄ (XIII)

dans laquelle R'₃ représente un atome d'hydrogène ou un radical méthyle, R'4 représente un radical alkyle linéaire ou ramifié comportant de huit à trente atomes de carbone et n' représente un nombre supérieur ou égal à un et inférieur ou égal à cinquante.

Comme exemples d'agents épaississants et/ou gélifiants que l'on peut associer au dit latex inverse auto-inversible tel que défini précédemment dans lesdites compositions (F) et (G), il y a les polysaccharides constitués uniquement d'oses, comme les glucanes ou homopolymères du glucose, les glucomannoglucanes, les xyloglycanes, les galactomannanes dont le degré de substitution (DS) des unités de D-galactose sur la chaîne principale de D-mannose est compris entre 0 et 1, et plus particulièrement entre 1 et 0,25, comme les galactomannanes provenant de la gomme de cassia (DS = 1/5), de la gomme de caroube (DS = 1/4), de la gomme de tara (DS = 1/3), de la gomme de guar (DS = 1/2), de la gomme de fenugrec (DS = 1 ).

Comme exemples d'agents épaississants et/ou gélifiants que l'on peut associer au dit latex inverse auto-inversible tel que défini précédemment dans lesdites compositions (F) et (G), il y a les polysaccharides constitués de dérivés d'oses, comme les galactanes sulfatés et plus particulièrement les carraghénanes et l'agar, les uronanes et plus particulièrement les algines, les alginates et les pectines, les hétéropolymères d'oses et d'acides uroniques et plus particulièrement la gomme xanthane, la gomme gellane, les exsudats de gomme de arabique et de gomme de karaya, les glucosaminoglycanes.

Comme exemples d'agents épaississants et/ou gélifiants que l'on peut associer au dit latex inverse auto-inversible tel que défini précédemment dans lesdites compositions (F) et (G), il y a la cellulose, les dérivés de cellulose comme la méthyl-cellulose, l'éthyl-cellulose, l'hydroxypropyl cellulose, les silicates, l'amidon, les dérivés hydrophiles de l'amidon, les polyuréthanes.

Comme exemples d'agents stabilisants que l'on peut associer au dit latex inverse auto-inversible tel que défini précédemment dans lesdites compositions (F) et (G), il y a les cires microcristallines, et plus particulièrement l'ozokérite, les sels minéraux tels que le chlorure de sodium ou le chlorure de magnésium, les polymères siliconés tels que les copolymères polysiloxane polyalkyl polyéther.

Comme exemples de solvants que l'on peut associer au dit latex inverse auto-inversible tel que défini précédemment dans lesdites compositions (F) et (G), il y a l'eau, les solvants organiques comme le glycérol, le diglycérol, les oligomères du glycérol, l'éthylène glycol, le propylène glycol, le butylène glycol, le 1,3-propanediol, le 1,2-propanediol, l'hexylèneglycol, le diéthylèneglycol, le xylitol, l'érythritol, le sorbitol, les alcools hydrosolubles tels que l'éthanol, l'isopropanol ou le butanol, les mélanges d'eau et desdits solvants organiques.

Comme exemples d'eaux thermales ou minérales que l'on peut associer au dit latex inverse auto-inversible tel que défini précédemment dans lesdites compositions (F) et (G), il y a les eaux thermales ou minérales ayant une minéralisation d'au moins 300 mg/l, en particulier l'eau d'Avene, l'eau de Vittel, les eaux du bassin de Vichy, l'eau d'Uriage, l'eau de la Roche Posay, l'eau de la Bourboule, l'eau d'Enghien-les-bains, l'eau de Saint-Gervais-les bains, l'eau de Néris-les-bains, l'eau d'Allevard-les-bains, l'eau de Digne, l'eau des Maizieres, l'eau de Neyrac-les-bains, l'eau de Lons le Saunier, l'eau de Rochefort, l'eau de Saint Christau, l'eau des Fumades et l'eau de Tercis-les-bains.

Comme exemples d'agents hydrotropes que l'on peut associer au dit latex inverse auto-inversible tel que défini précédemment, dans lesdites compositions (F) et (G), il y a les xylènes sulfonates, les cumènes sulfonates, l'hexylpolyglucoside, le 2-éthylhexylpolyglucoside, le n-heptylpolyglucoside.
Comme exemples d'agents tensioactifs émulsionnants que l'on peut associer au dit latex inverse auto-inversible tel que défini précédemment dans lesdites compositions (F) et (G), il y a les tensioactifs non ioniques, des tensioactifs anioniques, des tensioactifs cationiques.

Comme exemples de tensioactifs non-ioniques émulsionnants que l'on peut associer au dit latex inverse auto-inversible tel que défini précédemment dans lesdites compositions (F) et (G), il y a les esters d'acides gras et de sorbitol, comme les produits commercialisés sous les appellations MONTANE^{™}40, MONTANE^{™}60, MONTANE^{™}70, MONTANE^{™}80 et MONTANE^{™}85 ; les compositions comprenant du stéarate de glycérol et l'acide stéarique éthoxylé entre 5 moles et 150 moles d'oxyde d'éthylène, comme la composition comprenant de l'acide stéarique éthoxylé à 135 moles d'oxyde d'éthylène et du stéarate de glycérol commercialisée sous l'appellation SIMULSOL^{™} 165 ; les esters de mannitan ; les esters de mannitan éthoxylés ; les esters de sucrose ; les esters de méthylglucoside ; les alkylpolyglycosides comportant un radical aliphatique, linéaire ou ramifié, saturé ou insaturé, et comportant de 14 à 36 atomes de carbone, comme le tétradécyl polyglucoside, l'hexadécyl polyglucoside, l'octadécyl polyglucoside, l'hexadécyl polyxyloside, l'octadécyl polyxyloside, l'eicosyl polyglucoside, le dodécosyl polyglucoside, le 2-octyldodécyl polyxyloside, le 12-hydroxystéaryl polyglucoside ; les compositions d'alcools gras linéaires ou ramifiés, saturés ou insaturés, et comportant de 14 à 36 atomes de carbone, et d'alkylpolyglycosides tels que décrits précédemment, par exemple les compositions commercialisées sous les noms MONTANOV^{™}68, MONTANOV^{™}14, MONTANOV^{™}82, MONTANOV^{™}202, MONTANOV^{™}S, MONTANOV^{™}WO18, MONTANOV^{™}L, FLUIDANOV^{™}20X et EASYNOV^{™}.

Comme exemples de tensioactifs anioniques que l'on peut associer au dit latex inverse auto-inversible tel que défini précédemment dans lesdites compositions (F) et (G), il y a le glycéryl stéarate citrate, le cétéarylsulfate, les savons comme le stéarate de sodium ou le stéarate de triéthanolammonium, les dérivés N-acylés d'acides aminés salifiés, par exemple le stéaroyl glutamate.

Comme exemples de tensioactifs cationiques émulsionnants que l'on peut associer au dit latex inverse auto-inversible tel que défini précédemment dans lesdites compositions (F) et (G), il y a les aminoxydes, le quaternium-82 et les tensioactifs décrits dans la demande de brevet WO96/00719 et principalement ceux dont la chaîne grasse comprend au moins 16 atomes de carbone.

Comme exemples d'agents opacifiants et/ou nacrants que l'on peut associer au dit latex inverse auto-inversible tel que défini précédemment dans lesdites compositions (F) et (G), il y a le palmitate de sodium, le stéarate de sodium, l'hydroxystéarate de sodium, le palmitate de magnésium, le stéarate de magnésium, l'hydroxystéarate de magnésium, le monostéarate d'éthylène glycol, le distéarate d'éthylène glycol, le monostéarate de polyéthylène glycol, le distéarate de polyéthylène glycol, les alcools gras comportant de 12 à 22 atomes de carbone.

Comme exemples d'agents de texture que l'on peut associer au dit latex inverse auto-inversible tel que défini précédemment dans lesdites compositions (F) et (G), il y a des dérivés N-acylés d'acides aminés, comme la lauroyl lysine commercialisée sous l'appellation AMINOHOPE^{™}LL, l'octenyl starch succinate commercialisé sous l'appellation DRYFLO^{™}, le myristyl polyglucoside commercialisé sous l'appellation MONTANOV^{™} 14, les fibres de cellulose, les fibres de coton, les fibres de chitosane, le talc, la séricite, le mica Comme exemples d'agents déodorants que l'on peut associer au dit latex inverse auto-inversible tel que défini précédemment dans lesdites compositions (F) et (G), il y a les silicates alcalins, les sels de zinc comme le sulfate de zinc, le gluconate de zinc, le chlorure de zinc, le lactate de zinc ; les sels d'ammonium quaternaires comme les sels de cétyltriméthylammonium, les sels de cétylpyridinium ; les dérivés du glycérol comme le caprate de glycérol , le caprylate de glycérol, le caprate de polyglycérol ; le 1,2 décanediol ; le 1,3 propanediol ; l'acide salicylique ; le bicarbonate de sodium ; les cyclodextrines ; les zéolithes métalliques ; le TRICLOSAN^{™} ; le bromohydrate d'aluminium, les chlorhydrates d'aluminium, le chlorure d'aluminium, le sulfate d'aluminium, les chlorhydrates d'aluminium et de zirconium, le trichlorhydrate d'aluminium et de zirconium, le tétrachlorhydrate d'aluminium et de zirconium, le pentachlorhydrate d'aluminium et de zirconium, l'octochlorhydrate d'aluminium et de zirconium, le sulfate d'aluminium, le lactate de sodium et d'aluminium, les complexes de chlorhydrate d'aluminium et de glycol, comme le complexe de chlorhydrate d'aluminium et de propylène glycol, le complexe de dichlorhydrate d'aluminium et de propylène glycol, le complexe de sesquichlorhydrate d'aluminium et de propylène glycol, le complexe de chlorhydrate d'aluminium et de polyéthylène glycol, le complexe de dichlorhydrate d'aluminium et de polyéthylène glycol, le complexe de sesquichlorhydrate d'aluminium et de polyéthylène glycol.

Comme exemples d'huiles que l'on peut associer au dit latex inverse auto-inversible tel que défini précédemment dans lesdites compositions (F) et (G), il y a les huiles minérales telles que l'huile de paraffine, l'huile de vaseline, les isoparaffines ou les huiles blanches minérales ; les huiles d'origine animale, telles que le squalène ou le squalane ; les huiles végétales, telles que le phytosqualane, l'huile d'amandes douces, l'huile de coprah, l'huile de ricin, l'huile de jojoba, l'huile d'olive, l'huile de colza, l'huile d'arachide, l'huile de tournesol, l'huile de germes de blé, l'huile de germes de maïs, l'huile de soja, l'huile de coton, l'huile de luzerne, l'huile de pavot, l'huile de potiron, l'huile d'onagre, l'huile de millet, l'huile d'orge, l'huile de seigle, l'huile de carthame, l'huile de bancoulier, l'huile de passiflore, l'huile de noisette, l'huile de palme, le beurre de karité, l'huile de noyau d'abricot, l'huile de calophyllum, l'huile de sysymbrium, l'huile d'avocat, l'huile de calendula, les huiles issues de fleurs ou de légumes les huiles végétales éthoxylées ; les huiles synthétiques comme les esters d'acides gras tels que le myristate de butyle, le myristate de propyle, le myristate d'isopropyle, le myristate de cétyle, le palmitate d'isopropyle, le palmitate d'octyle, le stéarate de butyle, le stéarate d'hexadécyle, le stéarate d'isopropyle, le stéarate d'octyle, le stéarate d'isocétyle, l'oléate dodécyle, le laurate d'hexyle, le dicaprylate de propylèneglycol, les esters dérivés d'acide lanolique, tels que le lanolate d'isopropyle, le lanolate d'isocétyle, les monoglycérides, diglycérides et triglycérides d'acides gras comme le triheptanoate de glycérol, les alkylbenzoates, les huiles hydrogénées, les poly(alpha-oléfine), les polyoléfines comme le poly(isobutane), les isoalcanes de synthèse comme l'isohexadécane, l'isododécane, les huiles perfluorées ; les huiles de silicone comme les diméthylpolysiloxanes, les méthylphényl - polysiloxanes, les silicones modifiées par des amines, les silicones modifiés par des acides gras, les silicones modifiés par des alcools, les silicones modifiés par des alcools et des acides gras, des silicones modifiés par des groupements polyéther, des silicones époxy modifiés, des silicones modifiées par des groupements fluorés, des silicones cycliques et des silicones modifiées par des groupements alkyles. Par « huiles », on entend dans la présente demande les composés et/ou les mélanges de composés insolubles dans l'eau, se présentant sous un aspect liquide à une température de 25°C.

Comme exemples de cires que l'on peut associer au dit latex inverse auto-inversible tel que défini précédemment dans lesdites compositions (F) et (G), il y a la cire d'abeille, la cire de carnauba, la cire de candelilla, la cire d'ouricoury, la cire du Japon, la cire de fibre de liège, la cire de canne à sucre, les cires de paraffines, les cires de lignite, les cires microcristallines, la cire de lanoline ; l'ozokérite ; la cire de polyéthylène ; les cires de silicone ; les cires végétales ; les alcools gras et les acides gras solides à température ambiante ; les glycérides solides à température ambiante. Par « cires », on entend dans la présente demande les composés et/ou les mélanges de composés insolubles dans l'eau, se présentant sous un aspect solide à une température supérieure ou égale à 45°C.

Comme exemples de principes actifs que l'on peut associer au dit latex inverse auto-inversible tel que défini précédemment dans lesdites compositions (F) et (G), il y a les vitamines et leurs dérivés, notamment leurs esters, tels que le rétinol (vitamine A) et ses esters (palmitate de rétinyle par exemple), l'acide ascorbique (vitamine C) et ses esters, les dérives de sucre de l'acide ascorbique (comme l'ascorbyl glucoside), le tocophérol (vitamine E) et ses esters (comme l'acétate de tocophérol), les vitamines B3 ou B10 (niacinamide et ses dérivés) ; les composés montrant une action éclaircissante ou dépigmentante de la peau comme le w-undecelynoyl phénylalanine commercialisé sous l'appellation SEPIWHITE^{™}MSH, le SEPICALM^{™}VG, le mono ester et/ou le diester de glycérol du w-undecelynoyl phénylalanine, les ω-undecelynoyl dipeptides, l'arbutine, l'acide kojique, l'hydroquinone ; les composés montrant une action apaisante notamment le SEPICALM^{™} S, l'allantoïne et le bisabolol ; les agents anti-inflammatoires ; les composés montrant une action hydratante comme l'urée, les hydroxyurées, le glycérol, les polyglycérols, le glycérolglucoside, le diglycérolglucoside, les polyglycérylglucosides, le xylitylplucoside ; les extraits végétaux riches en polyphénols comme les extraits de raisin, les extraits de pin, les extraits de vin, les extraits d'olives ; les composés montrant une action amincissante ou lipolytique comme la caféine ou ses dérivés, l'ADIPOSLIM^{™}, l'ADIPOLESS^{™}, la fucoxanthine ; les protéines N-acylées ; les peptides N-acylés comme le MATRIXIL^{™} ; les acides aminés N-acylés ; les hydrolysâts partiels de protéines N-acylés ; les acides aminés ; les peptides ; les hydrolysâts totaux de protéines ; les extraits de soja, par exemple la Raffermine^{™} ; les extraits de blé par exemple la TENSINE^{™} ou la GLIADINE^{™} ; les extraits végétaux, tels que les extraits végétaux riches en tanins, les extraits végétaux riches en isoflavones ou les extraits végétaux riches en terpènes ; les extraits d'algues d'eau douce ou marines ; les extraits de plantes marines ; les extraits marins en général comme les coraux ; les cires essentielles ; les extraits bactériens ; les céramides ; les phospholipides ; les composés montrant une action antimicrobienne ou une action purifiante, comme le LIPACIDE^{™} C8G, le LIPACIDE^{™} UG, le SEPICONTROL^{™} A5 ; l'OCTOPIROX^{™} ou le SENSIVA^{™} SC50 ; les composés montrant une propriété énergisante ou stimulante comme le PHYSIOGENYL^{™}, le panthénol et ses dérivés comme le SEPICAP^{™} MP ; les actifs anti-âge comme le SEPILIFT^{™} DPHP, le LIPACIDE^{™} PVB, le SEPIVINOL^{™}, le SEPIVITAL^{™}, le MANOLIVA^{™}, le PHYTO-AGE^{™}, le TIMECODE^{™} ; le SURVICODE^{™} ; les actifs anti-photo vieillissement ; les actifs protecteurs de l'intégrité de la jonction dermo-épidermique ; les actifs augmentant la synthèse des composants de la matrice extracellulaire comme le collagène, les élastines, les glycosaminoglycanes ; les actifs agissant favorablement sur la communication cellulaire chimique comme les cytokines ou physiques comme les intégrines ; les actifs créant une sensation de « chauffe » sur la peau comme les activateurs de la microcirculation cutanée (comme les dérivés de l'acide nicotinique) ou des produits créant une sensation de « fraîcheur » sur la peau (comme le menthol et des dérivés) ; les actifs améliorant la microcirculation cutanée, par exemple les veinotoniques ; les actifs drainants ; les actifs à visée décongestionnante comme les extraits de ginko biloba, de lierre, de marron d'inde, de bambou, de ruscus, de petit houx, de *centalla asiatica*, de fucus, de romarin, de saule ; les agents de bronzage ou de brunissement de la peau, par exemple la dihydroxyacétone (DHA), l'érythrulose, l'aldéhyde mésotartrique, le glutaraldéhyde, le glycéraldéhyde, l'alloxane, la ninhydrine, les extraits végétaux par exemple les extraits de bois rouges du genre Pterocarpus et du genre Baphia comme le Pteropcarpus santalinus, le Pterocarpus osun, le Pterocarpus soyauxii, le Pterocarpus erinaceus, le Pterocarpus indicus ou le Baphia nitida comme ceux décrits dans la demande de brevet Européen EP 0 971 683 ; les agents connus pour leur action de facilitation et/ou d'accélération du bronzage et/ou du brunissement de la peau humaine, et/ou pour leur action de coloration de la peau humaine, par exemple les caraténoïdes ( et plus particulièrement le beta carotène et le gamma carotène), le produit commercialisé sous le nom de marque « Carrot oil » (Nom INCI : Daucus Carota, helianthus annuus Sunflower oil) par la société Provital, qui contient des caroténoïdes, de la vitamine E et de la vitamine K ; la tyrosine et/ou ses dérivés, connus pour leur effet sur l'accélération du bronzage de la peau humaine en association avec une exposition aux rayonnements ultra-violets, par exemple le produit commercialisé sous le nom de marque « SunTan Accelerator^{™} » par la société Provital qui contient de la tyrosine et des riboflavines (vitamine B), le complexe de tyrosine et de tyrosinase commercialisé sous le nom de marque « Zymo Tan Complex » par la société Zymo Line, le produit commercialisé sous le nom de marque MelanoBronze^{™} (nom INCI : Acetyl Tyrosine, Monk's pepper extract (Vitex Agnus-castus)) par la société Mibelle qui contient de l'acétyl tyrosine, produit commercialisé sous le nom de marque Unipertan VEG-24/242/2002 (nom INCI : butylene glycol and Acetyl Tyrosine and hydrolyzed vegetable protein and Adenosine triphosphate) par la société UNIPEX, le produit commercialisé sous le nom de marque « Try-Excell^{™} » (nom INCI : Oleoyl Tyrosine and Luffa Cylindrica (Seed) Oil and Oleic acid) par la société Sederma qui contient des extraits de pépins de courge (ou huile de Loofah), le produit commercialisé sous le nom de marque «Actibronze^{™} » (nom INCI : hydrolyzed wheat protein and acetyl tyrosine and copper gluconate) par la société Alban Muller, le produit commercialisé sous le nom de marque Tyrostan^{™} (nom INCI : potassium caproyl tyrosine) par la société Synerga, le produit commercialisé sous le nom de marque Tyrosinol (nom INCI : Sorbitan Isostearate, glyceryl oleate, caproyl Tyrosine) par la société Synerga, le produit commercialisé sous le nom de marque InstaBronze^{™} (nom INCI : Dihydroxyacetone and acetyl tyrosine and copper gluconate) commercialisé par la société Alban Muller, le produit commercialisé sous le nom de marque Tyrosilane (nom INCI : méthylsilanol and acétyl tyrosine) par la société Exymol ; les peptides connus pour leur effet d'activation de la mélanogénèse par exemple le produit commercialisé sous le nom de marque Bronzing SF Peptide powder (nom INCI : Dextran and Octapeptide-5) par la société Infinitec Activos, le produit commercialisé sous le nom de marque Melitane (nom INCI : Glycerin and Aqua and Dextran and Acetyl hexapeptide-1) comprenant l'acétyl hexapeptide-1 connu pour son action agoniste de l'alpha-MSH, le produit commercialisé sous le nom de marque Melatimes Solutions^{™} (nom INCI : Butylene glycol ,
Palmitoyl Tripeptide-40) par la société LIPOTEC, les sucres et les dérivés de sucres par exemple le produit commercialisé sous le nom de marque Tanositol^{™} (nom INCI : inositol) par la société Provital, le produit commercialisé sous le nom de marque Thalitan^{™} (ou Phycosaccharide^{™} AG) par la société CODIF international (nom INCI : Aqua and Hydrolyzed algin (Laminaria Digitata) and magnesium sulfate and manganese sulfate) contenant un oligosaccharide d'origine marine (acide guluronique et acide mannuronique chélatés avec les ions magnésium et manganèse), le produit commercialisé sous le nom de marque Melactiva^{™} (nom INCI : Maltodextrin, Mucuna Pruriens Seed extract) par la société Alban Muller, les composés riches en flavonoïdes par exemple le produit commercialisé sous le nom de marque « Biotanning » (nom INCI : Hydrolyzed citrus Aurantium dulcis fruit extract) par la société Silab et connu pour être riche en flavonoides de citron (de type hespéridines) ; les agents destinés au traitement des cheveux et/ou des poils, par exemple des agents protecteurs des mélanocytes du follicule pileux, destinés à protéger lesdits mélanocytes contre les agents cytotoxiques responsables de la sénescence et/ou de l'apoptose desdits mélanocytes, tels que les agents mimétiques de l'activité de la DOPAchrome tautomérase choisis parmi ceux décrits dans la demande de brevet européen publiée sous le numéro EP1515688 A2, les molécules synthétiques mimétiques de la SOD par exemples les complexes de manganèse, des composés antioxydants par exemple les dérivés de cyclodextrine, des composés silicés dérivés d'acide ascorbique, de la pyrrolidone carboxylate de lysine ou d'arginine, des associations de mono- et diester d'acide cinnamique et de vitamine C, et plus généralement ceux cités dans la demande de brevet européen publiée sous le numéro EP 1515 688 A2.
Comme exemples d'agents antioxydants que l'on peut associer au dit latex inverse auto-inversible tel que défini précédemment dans lesdites compositions (F) et (G), il y a l'EDTA et ses sels, l'acide citrique, l'acide tartarique, l'acide oxalique, le BHA (butylhydroxyanisol), le BHT (butylhydroxytoluène), les dérivés de tocophérol tels que l'acétate de tocophérol, des mélanges de composés antioxydants tels que la DISSOLVINE GL 47S commercialisé par la société Akzo Nobel sous le nom INCI : Tetrasodium Glutamate Diacetate.
Comme exemples de filtres solaires que l'on peut associer au dit latex inverse auto-inversible tel que défini précédemment dans lesdites compositions (F) et (G), il y a tous ceux figurant dans la directive cosmétique 76/768/CEE modifiée annexe VII.

Parmi les filtres organiques solaires que l'on peut associer au dit latex inverse auto-inversible tel que défini précédemment dans lesdites compositions (F) et (G), il y a la famille des dérivés de l'acide benzoïque comme les acides para-aminobenzoïques (PABA), notamment les esters de monoglycérol de PABA, les esters éthyliques de N,N25 propoxy PABA, les esters éthyliques de N,N-diéthoxy PABA, les esters éthyliques de N,Ndiméthyl PABA, les esters méthyliques de N,N-diméthyl PABA, les esters butyliques de N,Ndiméthyl PABA; la famille des dérivés de l'acide anthranilique comme l'homomenthyl-N-acétyl anthranilate ; la famille des dérivés de l'acide salicylique comme le salicylate d'amyle, le salicylate d'homomenthyle, le salicylate d'éthylhexyle, le salicylate de phényle, le salicylate de benzyle, le salicylate de p-isopropanolphényle ; la famille des dérivés de l'acide cinnamique comme le cinnamate d'éthylhexyle, le cinnamate d'éthyl-4-isopropyle, le cinnamate de méthyl- 2,5-diisopropyle, le cinnamate de p-méthoxypropyle, le cinnamate de p-méthoxyisopropyle, le cinnamate de p-méthoxyisoamyle, le cinnamate de p-méthoxyoctyle (le cinnamate de pméthoxy 2-éthylhexyle), le cinnamate de p-méthoxy 2-éthoxyéthyle, le p-cinnamate de méthoxycyclohexyle, le cinnamate d'éthyl-α-cyano-β-phényle, le cinnamate de 2-éthylhexyl-α-cyano-β-phényle, le cinnamate de diparaméthoxy mono-2-éthylhexanoyl de glycéryle ; la famille des dérivés de la benzophénone comme la 2,4-dihydroxybenzophénone, la 2,2'-dihydroxy-4-méthoxybenzophénone, la 2,2',4,4'-tétrahydroxybenzophénone, la 2-hydroxy-4-méthoxybenzophénone, la 2-hydroxy-4-méthoxy-4'-méthylbenzophénone, la 2-hydroxy-4-méthoxybenzophénone-5-sulfonate, la 4-phénylbenzophénone, le 2-éthylhexyl-4'-phénylbenzophénone-2-5 carboxylate, la 2-hydroxy-4-n-octyloxybenzophénone, la 4-hydroxy-3-carboxybenzophénone ; le 3-(4'-méthylbenzylidène)-d,l-camphre, le 3 (benzylidène)-d,lcamphre, le benzalkonium méthosulfate camphre ; l'acide urocanique, l'urocanate d'éthyle ; la famille des dérivés de l'acide sulfonique comme l'acide sulfonique 2-phénylbenzimidazole-5 et ses sels ; la famille des dérivés de la triazine comme l'hydroxyphényl triazine, l'éthylhexyloxyhydroxyphényl-4-méthoxyphényltriazine, le 2,4,6-trianillino-(p-carbo-2'-éthylhexyl-1'-oxy)-1,3,5-triazine, le 4,4-((6-(((1,1-diméthyléthyl) amino)carbonyl)phenyl)amino)-1,3,5-triazine-2,4-diyl diimino) bis-(2-éthylhexyl) ester de l'acide benzoïque, le 2-phényl-5-méthylbenzoxazole, le 2,2'-hydroxy-5- méthylphénylbenzotriazole, le 2-(2'-hydroxy-5'-t-octylphényl)benzotriazole, le 2-(2'-hydroxy-5'-méthyphényl)benzotriazole; la dibenzazine; le dianisoylméthane, le 4-méthoxy-4"-tbutylbenzoylméthane ; la 5-(3,3-diméthyl-2-norbornylidène)-3-pentan-2-one ; la famille des dérivés du diphénylacrylate comme le 2-éthylhexyl-2-cyano-3,3-diphényl-2-propènoate, l'éthyl-2-cyano-3,3-diphényl-2-propènoate ; la famille des polysiloxanes comme le malonate de benzylidène siloxane.
Parmi les filtres inorganiques solaires, également appelés "écrans minéraux", que l'on peut associer au dit latex inverse auto-inversible tel que défini précédemment dans lesdites compositions (F) et (G), il y a les oxydes de titane, les oxydes de zinc, l'oxyde de cérium, l'oxyde de zirconium, les oxydes de fer jaune, rouge ou noir, les oxydes de chrome. Ces écrans minéraux peuvent être micronisés ou non, avoir subi ou non des traitements de surface et être éventuellement présentés sous formes de pré-dispersions aqueuses ou huileuses.

L'exemple suivant illustre l'invention, sans toutefois la limiter.

### 1- Exemples

1.1 Préparation d'un latex inverse (LI₁) comprenant un copolymère réticulé du sel de sodium de l'acide 2-méthyl-[(1-oxo-2-propenyl)amino] 1-propanesulfonique et de l'acrylamide contenant l'éthylènediamine disuccinique acide sous forme de sel trisodique comme agent séquestrant.

On charge dans un bécher sous agitation :
- 272 g d'une solution commerciale à 55% de sel de sodium de l'acide 2-méthyl- [(1-oxo-2-propenyl)amino] 1-propanesulfonique
- 255 g d'une solution à 50% d'acrylamide
- 115 g d'acide 2-méthyl- [(1-oxo-2-propenyl)amino] 1-propanesulfonique
- 0,115 g de méthylène bis-acrylamide
- 0,62 g d'une solution commerciale d'ethylenediamine disuccinique acide sous forme de sel trisodique
- 0,14 g de sulfate de cuivre pentahydraté

Le pH de la phase aqueuse est ajusté à 6 avec une solution de soude 48%. La phase organique est préparée en parallèle en mélangeant :
- 220 grammes d'isohéxadécance
- 21 grammes Montane^{™} 80 ⁽¹⁾

(1) : Montane^{™}80 est un monooléate de sorbitan, tensioactif émulsionnant de type eau-dans-huile, commercialisé par la société SEPPIC.
   La phase aqueuse préparée ci-dessus est ajoutée progressivement sur la phase huileuse puis dispersée à l'aide d'un rotor stator type Ultra Turrax^{™} commercialisé par la société Ika^{™}.
   L'émulsion obtenue est ensuite transférée dans un réacteur double-enveloppé, et soumise à un barbotage d'azote pour éliminer l'oxygène. Une solution à 0,56% en poids d'hydropéroxyde de cumène dans 5 mL d'isohéxadécane est introduite et l'émulsion est maintenue sous agitation pendant 5 minutes d'homogénéisation à température ambiante.
   La réaction de polymérisation est initié en ajoutant 15 mL d'une solution aqueuse contenant 0,2% de métabisulfite de sodium. Une fois la réaction de polymérisation terminée, le milieu réactionnel est chauffé à 85°C pendant 1h puis l'ensemble est refroidi jusqu'à environ 35°C, ensuite 50 g de Montanox^{™} 80 ⁽²⁾ sont ajoutés sur la préparation.
(2) : Montanox^{™}80 est un dérivé polyoxyéthyléné d'un monooléate de sorbitan, utilisé comme tensioactif de type huile-dans-eau, commercialisé par la société SEPPIC.

Le produit obtenu est référencé (LI₁) et les résultats de ses évaluations sont présentés dans le Tableau 1.

1.2 Préparation d'un latex inverse (LI₂) comprenant un copolymère réticulé du sel de sodium de l'acide 2-méthyl-[(1-oxo-2-propenyl)amino] 1-propanesulfonique et et de l'acrylamide contenant le diéthylènetriamine pentaacétate de sodium comme agent séquestrant.

Le même protocole que l'exemple 1.1 est mis en œuvre mais les 0,62 g d'une solution commerciale d'éthylènediamine disuccinique acide sous forme de sel trisodique sont substitués par 0.45 g d'une solution de diéthylènetriamine pentaacétate de sodium (commercialisé sous le nom de marque Versenex^{™} 80).

Le produit est référencé (LI₂).

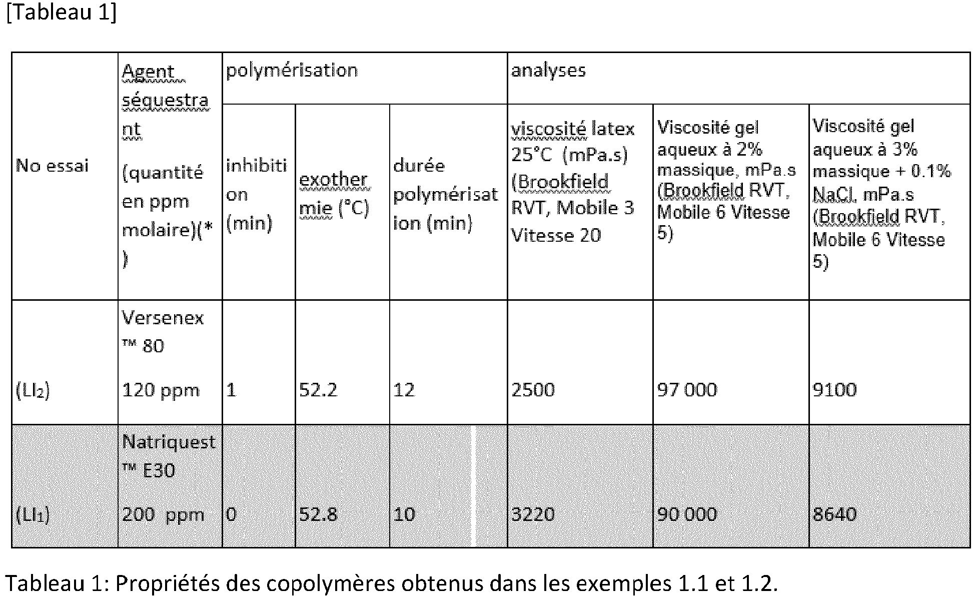

En conclusion, les essais de copolymérisation de l'acide 2-méthyl-[(1-oxo-2-propenyl)amino] 1-propanesulfonique et de l'acrylamide montrent qu'en présence de cations de cuivre, le sel trisodique d'éthylènediamine disuccinique acide présente une efficacité similaire au diéthylènetriamine pentaacétate de sodium.

Dans chacun des essais, la polymérisation présente des caractéristiques semblables: durée d'inhibition, de polymérisation et exothermie. Les latex inverses auto-inversibles obtenus dans ces conditions possèdent des propriétés épaississantes équivalentes dans l'eau et en présence d'électrolytes.

## Revendications

1. Procédé de préparation d'un latex inverse auto-inversible comprenant une phase aqueuse comprenant :
a. Un polyélectrolyte anionique réticulé (P) constitué pour 100% molaire de :
- Une proportion entre 20% et 90% d'une unité monomérique issue de l'acide 2-méthyl-2-[(1-oxo-2-propényl) amino] 1-propanesulfonique sous forme d'acide libre ou partiellement ou totalement salifiée ;
- Une proportion entre 10% et 80% d'une unité monomérique issue d'au moins un monomère choisi parmi les éléments du groupe constitué par l'acrylamide, le N,N-diméthyl acrylamide, le méthacrylamide, le N-isopropyl acrylamide, le N-tertio-butyl acrylamide ; et
- Une proportion supérieure à 0% molaire et inférieure ou égale à 1% molaire d'une unité monomérique issue d'au moins un monomère de réticulation (AR) polyéthylénique choisi parmi le méthylène-bis(acrylamide), le diméthacrylate d'éthylène glycol, le diacrylate de diéthylèneglycol, le diacrylate d'éthylène glycol, le diallyl urée, le triallylamine, le triméthylol propanetriacrylate, l'acide diallyoxyacétique ou un de ses sels comme le diallyloxyacétate de sodium, ou un mélange de ces composés,
b. De l'éthylenediamine disuccinique acide sous forme de sel trisodique,
ledit procédé comprenant les étapes suivantes :
a) Préparation de la phase aqueuse telle que définie ci-dessus,
b) Préparation d'une phase organique comprenant au moins une huile et un système tensioactif émulsionnant (S₁) de type eau-dans-huile,
c) Mélange de la phase aqueuse et de la phase organique préparées aux étapes a) et b) et émulsification de manière à former une émulsion,
d) Inertage de l'émulsion à l'azote,
e) Amorçage de la réaction de polymérisation par introduction dans l'émulsion inertée d'un initiateur de radicaux libres, et
f) Introduction dans le milieu réactionnel issu de l'étape e) d'un système tensioactif émulsionnant (S₂) de type huile-dans-eau à une température comprise entre 30 et 60°C.

2. Procédé selon la revendication 1, **caractérisé en ce que** la phase aqueuse dudit latex inverse auto-inversible comprend au moins 0,01% molaire de l'éthylenediamine disuccinique acide sous forme de sel trisodique.

3. Procédé selon l'une des revendications 1 ou 2, **caractérisé en ce que** le monomère de réticulation (AR) présent dans ladite phase aqueuse est du méthylène bis(acrylamide) ou du triallylamine.

4. Procédé selon l'une des revendications 1à 3, **caractérisé en ce qu'**à l'étape e) l'initiateur de radicaux est un couple oxydo-réducteur générateur d'ions hydrogénosulfite (HSO3-), tel que le couple hydropéroxyde de cumène -métabisulfite de sodium (Na2S2O5) ou le couple hydroperoxyde de cumène-chlorure de thionyle (SOCl2).

5. Procédé selon l'une des revendications 1 à 4, **caractérisé en ce qu'**à l'étape e) on introduit dans l'émulsion inertée un co-initiateur de polymérisation, de préférence de l'azo-bis(isobutyronitrile).

6. Procédé selon l'une des revendications 1 à 5, **caractérisé en ce qu'**à l'étape a) le pH de la phase aqueuse est ajusté entre 3,0 et 7,0.

7. Procédé selon l'une des revendications 1 à 6, **caractérisé en ce que** le milieu réactionnel issu de l'étape e) est concentré par distillation avant la mise en œuvre de l'étape f).

8. Procédé selon l'une des revendications 1 à 7, **caractérisé en ce que** le milieu réactionnel issu de l'étape e) ou f) est séché par atomisation.

## Patentansprüche

1. Verfahren zur Herstellung eines selbstumkehrbaren inversen Latex, der eine wässrige Phase umfasst, umfassend:
a. Ein vernetztes anionisches Polyelektrolyt (P), das zu 100 Mol-% aus folgenden Bestandteilen besteht:
- einem Anteil zwischen 20 % und 90 % einer Monomereinheit, die von 2-Methyl-2-[(1-oxo-2-propenyl)amino]1-propansulfonsäure in Form von freier Säure oder teilweise oder vollständig salzbildender Säure abgeleitet ist;
- einem Anteil zwischen 10 % und 80 % einer Monomereinheit, die aus mindestens einem Monomer stammt, das aus der Gruppe ausgewählt ist, die aus Acrylamid, N,N-Dimethylacrylamid, Methacrylamid, N-Isopropylacrylamid und N-tert-Butylacrylamid besteht; und
- Ein Anteil von mehr als 0 Mol-% und weniger als oder gleich 1 Mol-% einer Monomereinheit, die aus mindestens einem polyethylenischen Vernetzungsmonomer (AR) stammt, ausgewählt aus Methylenbis(acrylamid), Ethylenglykoldimethacrylat, Diethylenglykoldiacrylat, Ethylenglykoldiacrylat, Diallylharnstoff, Triallylamin, Trimethylolpropantriacrylat, Diallyoxyessigsäure oder eines ihrer Salze wie Natriumdiallyloxyacetat oder einer Mischung dieser Verbindungen,
b. Ethylendiamindisuccinat in Form von Trinatriumsalz,
wobei das Verfahren die folgenden Schritte umfasst:
a) Herstellung der wässrigen Phase, wie oben definiert,
b) Herstellung einer organischen Phase, die mindestens ein Öl und ein emulgierendes Tensidsystem (S₁ ) vom Typ Wasser-in-Öl umfasst,
c) Mischen der in den Schritten a) und b) hergestellten wässrigen Phase und der organischen Phase und Emulgieren, um eine Emulsion zu bilden,
d) Inertisierung der Emulsion mit Stickstoff,
e) Auslösen der Polymerisationsreaktion durch Einbringen eines Radikalinitiators in die inertisierte Emulsion und
f) Einbringen eines emulgierenden Tensidsystems (S₂ ) vom Typ Öl-in-Wasser bei einer Temperatur zwischen 30 und 60 °C in das aus Schritt e) resultierende Reaktionsmedium.

2. Verfahren nach Anspruch 1, **dadurch gekennzeichnet, dass** die wässrige Phase des selbstumkehrbaren inversen Latex mindestens 0,01 Mol-% Ethylendiamindisuccinat in Form von Trinatriumsalz enthält.

3. Verfahren nach einem der Ansprüche 1 oder 2, **dadurch gekennzeichnet, dass** das in der wässrigen Phase vorhandene Vernetzungsmonomer (AR) Methylenbis(acrylamid) oder Triallylamin ist.

4. Verfahren nach einem der Ansprüche 1 bis 3, **dadurch gekennzeichnet, dass** in Schritt e) der Radikalinitiator ein Oxidations-Reduktions-Paar ist, das Hydrogensulfit-Ionen (HSO3-) erzeugt, wie beispielsweise das Paar Cumolhydroperoxid-Natriummetabisulfit (Na2S2O5) oder das Paar Cumolhydroperoxid-Thionylchlorid (SOCl2).

5. Verfahren nach einem der Ansprüche 1 bis 4, **dadurch gekennzeichnet, dass** in Schritt e) ein Polymerisations-Co-Initiator, vorzugsweise Azobis(isobutyronitril), in die inertisierte Emulsion eingebracht wird.

6. Verfahren nach einem der Ansprüche 1 bis 5, **dadurch gekennzeichnet, dass** in Schritt a) der pH-Wert der wässrigen Phase auf 3,0 bis 7,0 eingestellt wird.

7. Verfahren nach einem der Ansprüche 1 bis 6, **dadurch gekennzeichnet, dass** das aus Schritt e) stammende Reaktionsmedium vor der Durchführung von Schritt f) durch Destillation konzentriert wird.

8. Verfahren nach einem der Ansprüche 1 bis 7, **dadurch gekennzeichnet, dass** das aus Schritt e) oder f) stammende Reaktionsmedium durch Sprühtrocknung getrocknet wird.

## Claims

1. A process for preparing a self-reversible inverse latex comprising an aqueous phase comprising:
a. A cross-linked anionic polyelectrolyte (P) consisting of 100 mol% of:
- Between 20% and 90% of a monomeric unit derived from 2-methyl-2-[(1-oxo-2-propenyl)amino] 1-propanesulfonic acid in the form of free acid or partially or totally saltified;
- Between 10% and 80% of a monomeric unit derived from at least one monomer chosen from the group consisting of acrylamide, N,N-dimethyl acrylamide, methacrylamide, N-isopropyl acrylamide, N-tert-butyl acrylamide; and
- A proportion greater than 0 mol% and less than or equal to 1 mol% of a monomeric unit derived from at least one polyethylene cross-linking monomer (AR) selected from methylene bis(acrylamide), ethylene glycol dimethacrylate, diethylene glycol diacrylate, ethylene glycol diacrylate, diallyl urea, triallylamine, trimethylol propanetriacrylate, diallyloxyacetic acid or one of its salts such as sodium diallyloxyacetate, or a mixture of these compounds,
b. Ethylenediamine disuccinic acid in the form of trisodium salt,
said process comprising the following steps:
a) Preparation of the aqueous phase as defined above,
b) Preparation of an organic phase comprising at least one oil and a water-in-oil emulsifying surfactant system (S₁),
c) Mixing the aqueous phase and the organic phase prepared in steps a) and b) and emulsifying to form an emulsion,
d) Inerting the emulsion with nitrogen,
e) Initiating the polymerisation reaction by introducing a free radical initiator into the inerted emulsion, and
f) Introducing an oil-in-water emulsifying surfactant system (S₂) into the reaction medium resulting from step e) at a temperature between 30 and 60°C.

2. Process according to claim 1, **characterised in that** the aqueous phase of said self-reversible inverse latex comprises at least 0.01 mol% of ethylenediamine disuccinic acid in the form of trisodium salt.

3. Process according to one of claims 1 or 2, **characterised in that** the cross-linking monomer (AR) present in said aqueous phase is methylene bis(acrylamide) or triallylamine.

4. Process according to one of claims 1 to 3, **characterised in that** in step e) the radical initiator is an oxidising-reducing pair generating hydrogen sulphite ions (HSO3-), such as the cumene hydroperoxide-sodium metabisulphite (Na₂S₂O₅) or the cumene hydroperoxide-thionyl chloride (SOCl₂) couple.

5. Process according to one of claims 1 to 4, **characterised in that** in step e) a polymerisation co-initiator, preferably azo-bis(isobutyronitrile), is introduced into the inerted emulsion.

6. Process according to one of claims 1 to 5, **characterised in that** in step a) the pH of the aqueous phase is adjusted to between 3.0 and 7.0.

7. Process according to one of claims 1 to 6, **characterised in that** the reaction medium from step e) is concentrated by distillation before step f) is carried out.

8. Process according to one of claims 1 to 7, **characterised in that** the reaction medium resulting from step e) or f) is dried by atomisation.
